# EUROPEAN PATENT SPECIFICATION

(11) **EP 1 554 984 B1**
(45) Date of publication and mention of the grant of the patent: **18.06.2008**
(21) Application number: 05008396.3
(22) Date of filing: 01.10.1997
(51) Int. Cl.: A61B 17/128

(54) **Single-fire ligation clip module**
Abbindeklammer-Modul mit Einzelauslösung
Module de clip de ligature à déclenchement unique

(30) Priority: 08.10.1996 US 28121 P
(43) Date of publication of application: 20.07.2005
(62) Divisional of application: 97911625.8
(73) Proprietor: Covidien AG, 8212 Neuhausen am Rheinfall (CH)
(72) Inventor: Rousseau, Robert A., New Milford, CT 06776 (US); Corrao, Ernest N. Jr., Bethel, CT 06801 (US)
(74) Representative: HOFFMANN EITLE

(56) References cited:
- EP-A- 0 609 612
- US-A- 4 590 937
- US-A- 5 306 283
- US-A- 5 354 304

## Description

### TECHNICAL FIELD

The present invention relates generally to a medical instrument for use with a surgical ligation clip applicator. More particularly, the invention is directed to a unitary single-fire module for housing a two-part ligation clip for securing and closing the two-part ligation clip around a vessel to be occluded.

### BACKGROUND ART

A surgical ligation clip is used to compress a severed blood vessel to stop the flow of blood. The single-fire module of the subject invention is particularly designed for housing and applying a two-part surgical ligation clip. The first part comprises generally a Y-shaped clamp or track having two extended arms for fitting around a vessel to be ligated. The second part is a U-shaped clip body with an extended slot for slidably engaging the track and closing the arms of the clip track about the vessel. Ligation clips of this type are disclosed in U.S. Patent No. 4,590,937 and in U.S. Patent No. 5,306,283.

There are many types of known modules for use with mechanical applicators for closing a conventional ligation clip around a vessel. The module can be attached to the distal end of the mechanical applicator by a spring loaded latch as disclosed in U.S. Patent No. 5,354,304 or other type of latching means which attachably secures the module to the applicator. Several examples of other known applicators include the so-called push-pull type, which uses a plunger or a piston, or a fulcrum type applicator having scissors-like or pliers-like handle. EP 0 609 612 discloses a clip and an instrument with 'jaws' which act to close the clip, U.S. Patent No. 5,354,304 discloses a preferred single-fire mechanical applicator for use with the subject invention.

U.S. Patent No. 5,354,304 further discloses a prior art single-fire module for housing the two-part ligation clip prior to application. The prior art module holds the clip track of the ligation clip in a stationary position as an advancing pusher at the proximal end of the module moves the sliding clip body of the ligation clip forward in order to compress the clip track around a vessel of a patient. The prior art module comprises two substantially identical cartridge halves that are joined together by integral alignment pins that align the two halves together so that the two halves may be welded securely together during manufacturing. Upper and lower metal latches, each having an integral spring and opposing trunions, are housed inside the middle portion of the module and pivot in holes formed in each cartridge halve. The integral springs press against ribs also formed inside each cartridge halve and urge the proximal ends of the metal latches toward each other so that the metal latches are compressively housed inside the module. A prior art pusher is also provided at a proximal end opening of the module for actuating the two-part ligation clip when the applicator is actuated by the user. The latches are also provided with a V-shaped end portion which engages opposing grooves formed on either side of the prior art pusher for retaining the pusher inside the cartridge housing.

Although the above prior art module provides an efficient and advantageous means of occluding a vessel with a two-part ligation clip, several disadvantages remain. The prior art module is costly to manufacture because of the different parts that must be manufactured and put together during assembly. Specifically, the metal latches with their cantilevered compression springs are costly to manufacture and time-consuming to assemble into the prior module since this must be done manually. Further, the prior art module can tend to jam due to the metal latches being improperly assembled or defects that occur during manufacturing of the metal latches. Finally, the prior art module has a wide profile along its longitudinal axis which may block or interfere with the user's view of the vessel during application of the two-part clip, with the applicator.

There therefore exists a need in the art for a unitary single-piece module for housing and applying a ligation clip in which the module is of a single unitary design with no separate parts therein. There also exists a need in the art for a single-piece module that is less costly to manufacture and requires little or no assembly during manufacturing. Finally, there also exists a need for a single-piece module housing having a low profile that permits clearer viewing of a vessel during ligation.

### DISCLOSURE OF INVENTION

Accordingly, it is a general object of the present invention to provide an improved ligation clip module for housing a ligation clip.

It is another object of the present invention to provide a module for housing a two-part ligation clip, and more particularly, two-part ligation clips of the type disclosed in U.S. Patent Nos. 4,590,937 and 5,306,283.

It is still another object of the present invention to provide a module for attachment to a ligation clip applicator, and more particularly, a ligation clip applicator of the type disclosed in U.S. Patent No. 5,354,304.

It is still another object of the present invention to provide a module housing of unitary design and manufacture that requires no manual assembly during manufacturing and utilizes less parts to manufacture.

It is still another further object of the present invention to provide a module that is less costly to manufacture.

It is still another object of the present invention to provide a module that does not jam during application of the ligation clip to a vessel during ligation.

It is yet another object of the present invention to provide a module that forms a low profile for better viewing along its longitudinal axis.

In accordance with one aspect of the invention, a module for mounting a two-part ligation clip comprises a unitary module housing, a holding means formed as part of the cartridge housing for holding a first part of the ligation clip in a stationary position, and a sliding means, also housed in the cartridge housing, for advancing a second part of the ligation clip about the first part during application.

The subject invention thus provides a module for mounting the ligation clip onto an applicator. The module is preferably made primarily of a hard resilient plastic or other similar low cost material and is disposable. In addition, different size modules for mounting various size ligation clips can be interchangeably connected to the same, reusable applicator. The module is connected to a slender, elongated hollow shaft that houses a slidable advancer. A handle assembly connected to the other end of the shaft actuates the advancer to slide it axially within the shaft and engage a pusher disposed in the module. The module holds the clip track of the ligation clip in a stationary position as the advancing pusher moves the sliding clip body forwardly to compress the clip track around a vessel.

These and other objects, aspects, features and advantages of the present invention will become apparent from the following detailed description of the preferred embodiments taken in conjunction with the accompanying drawings.

### BRIEF DESCRIPTION OF DRAWINGS

FIG. **1** is an exploded perspective view of a prior art ligation clip module;
FIG. **2** is an exploded perspective view of the ligation clip module according to the present invention;
FIG. **3** is a side view perspective of the module housing according to the present invention;
FIG. **4** is a top view perspective of the module housing according to the present invention;
FIG. **5** is a rear view perspective of the module housing according to the present invention;
FIG. **6** is a cross section view of the module housing according to the present invention;
FIG. **7** is a perspective view of the ligation clip module according to the present invention along with a partial perspective of the distal end of a prior art ligation clip applicator;
FIG. **8** is a side elevational view, partly in vertical cross-section, of prior art ligation clip applicator illustrating how the ligation clip module according to the present invention is attached to the neck portion of the applicator;
FIG. **9** is a cross section view of the ligation clip module showing a perspective view of the ligation clip and pusher in the initial position after assembly according to the present invention;
FIG. **10** is a cross section view of the ligation clip module showing a perspective view of the ligation clip and pusher with the latch ramps engaged with the radiused surface of the pusher according to the present invention;
FIG. **11** is a cross section view of the ligation clip module showing a perspective view of the ligation clip and pusher with the ligation clip fully assembled and released according to the present invention;
FIG. **12** is a cross section view of the ligation clip module showing the perspective view of the ligation clip and pusher with the module in the "fire-thru" position according to the present invention.

### BEST MODE FOR CARRYING OUT THE INVENTION

As noted above, the single-fire ligation clip module **23** of the present invention is specifically designed to house and apply a two-part ligation clip of the types described in U.S. Patent Nos. 4,590,937 and 5,306,283. For convenience, however, the invention will be described with reference to the two-part ligation clip described in U.S. Patent No. 5,306,283. Further, the module **23** of the present invention can be used with the ligation clip applicator described in U.S. Patent No. 5,354,304.

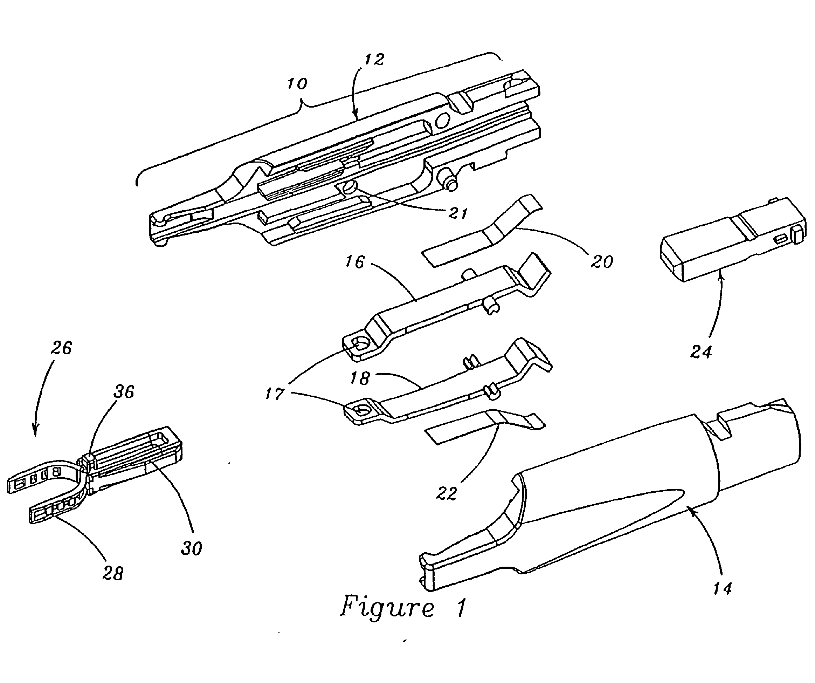

A prior art single-fire ligation clip module is shown in FIG. **1** and comprises a cartridge housing **10** that includes substantially identical first and second cartridge halves **12** and **14** that are joined together by engaging the integral alignment pins (not shown) in one cartridge half and welding or otherwise securing the halves **12** and **14** together. First and second latches **16** and **18** each have an first and second integral springs **20** and **22,** respectively, and opposing trunions **19** which pivot in holes **21** in both cartridge halves **12** and **14.** First and second integral springs **20** and **22** press against the interior surface of each cartridge half and urge the proximal ends of the latches **16** and **18** toward each other. First and second latches **16** and **18** further include latch holes **17** at its distal end which are connected to a post **36** of the ligation clip **26.**

Ligation clip **26** comprises a U-shaped clip body **30** slidably connected to a Y-shaped clip track **28** through a post **36** located at the apex of the clip track **28.** The post **36** extends and attaches to holes **17** of the first and second latches **18** and **20.** The attachment of holes **17** to the post **36** keeps the clip track **28** stationary when the clip track **28** is being drawn into the clip body **30** during application.

A prior art pusher **24** rides a track provided in the opposing cartridge halves **12** and **14.** The pusher **24** abuts against the proximal end of the clip body **30** in the initial assembled condition inside the cartridge housing **10** and is used to force the clip body **30** over the clip track **28** when actuated by the ligation clip applicator **92** during ligation.

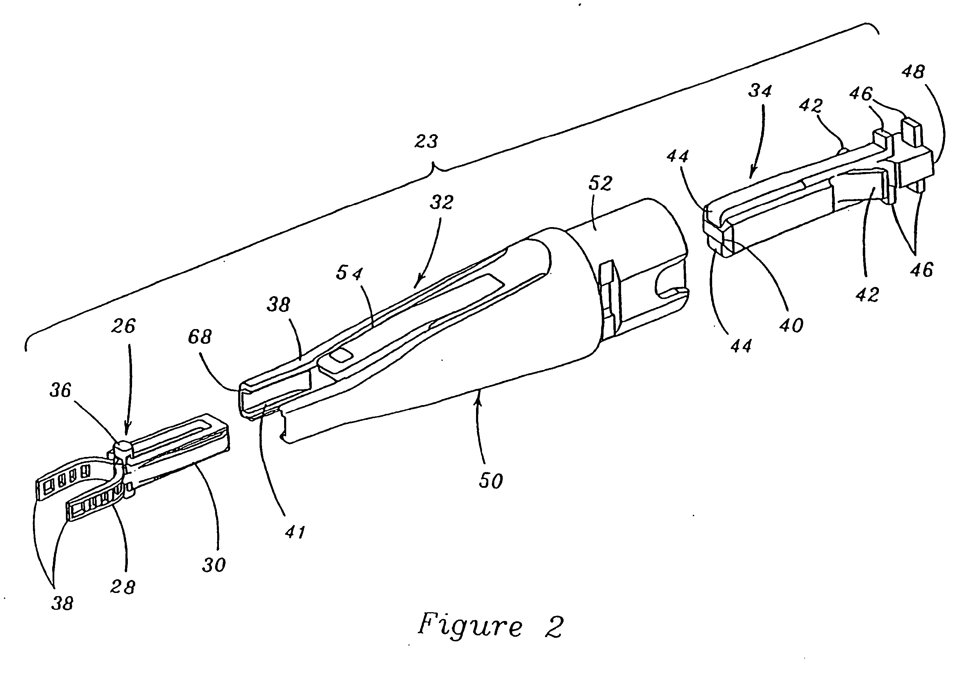

A module **23** according to the present invention is shown in FIG. **2****.** Module **23** comprises a unitary module housing **32** that comprises a frustoconical section **50** and a reduced diameter section **52.** During manufacturing, a pusher **34** is placed into a cavity **65** at the proximal end of the module housing **32.** Pusher **34** comprises a beveled front end **40** at its distal end and a rear portion **48** at its proximal end. Opposing radius surfaces **44** are formed on the top and bottom portions of pusher **34** while opposing wings **42** extend laterally at an acute angle from the side portions of the pusher **34.** Further, two pairs of flanges **46** located at the proximal end of pusher **34** extend from the top and bottom of the pusher **34.** Finally, a ligation clip **26** as described above is attached to the distal end of the pusher **26** during manufacturing.

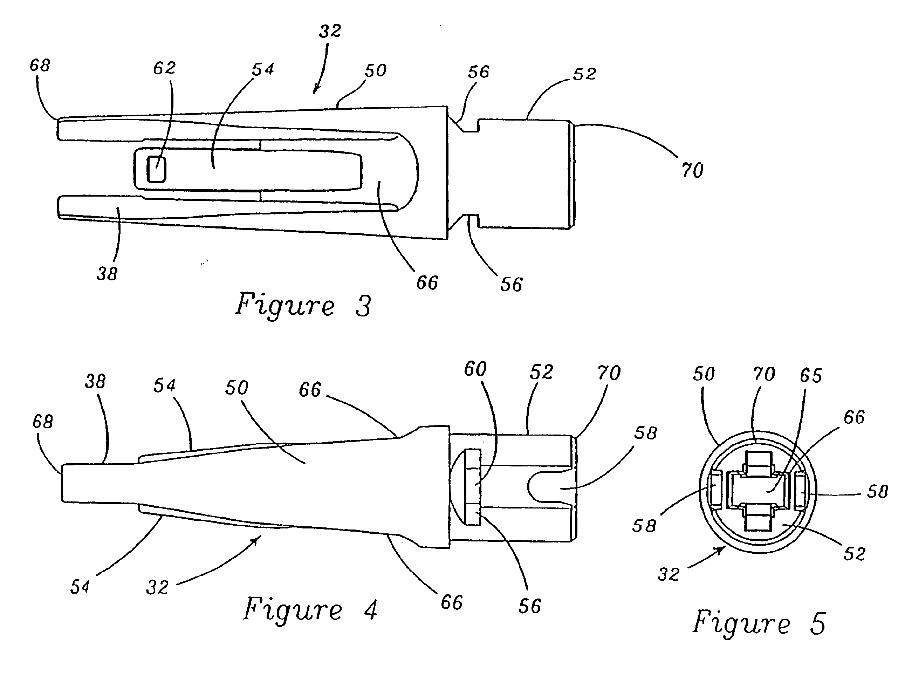

A more detailed description of the module housing **32** is shown in FIGS. **3-5****.** Module housing **32** is of a unitary construction in contrast to the two-part construction of the prior art cartridge housing **10.** Module housing **32** is preferably made of polycarbonate, however any hard plastic material suitable for housing and applying a ligation clip is felt to fall within the scope of the present invention.

As shown in FIG. **3****,** the frustoconical section **50** of module housing **32** has opposing tapered beveled portions **66** that each form an opposing latch **54** with the distal end of each latch **54** forming a hole **62** thereto. Preferably, the module housing **32** has two opposing latches **54,** although in alternative embodiments the housing **32** may have one latch or more than two latches. The distal end of frustoconical section **50** also forms opposing extending arms **38** that are in perpendicular relation to the latches **54.** As seen back in FIG. **2****,** the distal end of extended arms **38** include opposing tips **68** that form flanges that produces a channel **41.** The channel **41** serves to protect the clip track **28** seated therein during application of the ligation clip **26.** The reduced diameter section **52** of module housing **32** has a beveled rear end **70** and two opposing transverse grooves **56** that are diametrically opposed to one another along the distal portion of the reduced diameter section **52.** The transverse grooves **56** are used to attach the module **23** to a ligation clip applicator **92,** as shall be explained in greater detail below.

As can be seen in FIG. **4****,** a side view of the module housing **32** shows the opposing latches **54** of the frustoconical section **50** having a higher profile than the opposing beveled portions **66.** The frustoconical section **50** has a shape that gradually tapers along substantially its entire length towards its distal end. This gradual tapering of the frustoconical section **50** in combination with beveled portions **66** permits clearer viewing along the longitudinal axis of the module **23** looking towards its distal end since the beveled portions **66** cut away portions of section **50** that would normally obstruct viewing along that particular axis.

The opposing transverse grooves **56** of the reduced diameter section **52** each include a window **60** that extends into the cavity **65** of the module housing **32.** Diametrically opposing slot keys **58** are formed on the outer surface of the reduced diameter section **52** and are used to properly orient the module **23** during attachment of the module **23** to the ligation clip applicator **92.**

As shown in FIG. **5****,** the distal end of module housing **32** shows the slot keys **58** in diametrically opposed positions around the reduced diameter section **52.** A rear opening **64** is interposed between the two keys **58** and has a cross-like configuration that leads into the cavity **65** inside the module housing **32.** The cross-like configuration of rear opening **64** is shaped to receive the pusher **34** during assembly of the module **23,** wherein the pusher **34** is inserted into the rear opening **64** until opposing wings **42** of pusher **34** engage respective opposing windows **60** in the module housing **32.**

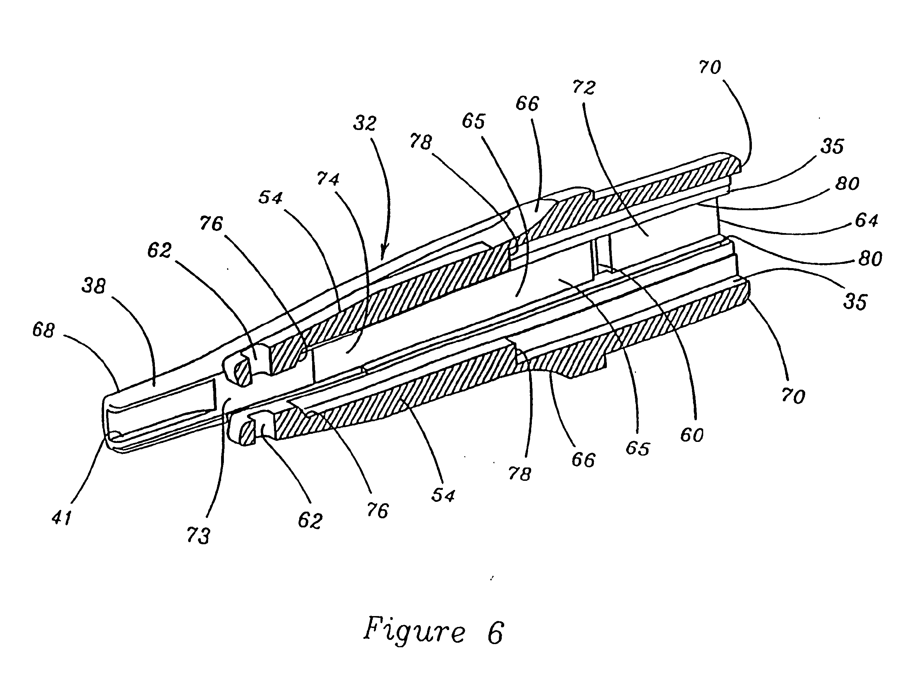

As can be seen in FIG. **6****,** the cavity **65** forms a first larger channel **72** towards the rear opening **64** of the module housing **32** which is in communication with a second smaller channel **74** at the distal end of housing **32.** First larger channel **72** includes a raceway **80** that slidably engages the opposing wings **42** of the pusher **34** and a track **35** that also slidably engages the flanges **46** of the pusher **34.** Opposing windows **60** are provided at the sidewalls of the reduced diameter section **52** and form an opening that permits viewing of the cavity **65** from diametrically opposed positions around the reduced diameter section **32.** During assembly of the module **23,** the pusher **34** is inserted into the rear opening **64** of the module housing **32** until the opposing wings **42** engage respective opposing windows **60.** In this orientation, the opposing wings **42** prevent any rearward movement of the pusher **34** so that the pusher **34** cannot be withdrawn form the module housing **32** through the rear opening **64.** Further, the junction between the first larger channel **72** and the second smaller channel **74** forms a shoulder **78** that is designed to abut the flanges **46** of the pusher **34** during application of the ligation clip **36** and prevent any further forward movement of the pusher **34** therethrough.

The integral latches **54** form the top and bottom portions of the second smaller channel **74** while the extending arms **38** of the frustoconical section **52** form the side portions of channel **74.** As shown back in FIG. **2****,** slits **55** separate the side and top and bottom portions of the second smaller channel **74** formed between the integral latches **54** and the extended arms **38.** The interior portion of the second smaller channel **74** formed by the integral latches **54** also include opposing latch ramps **76** that further restrict the size of channel **74** leading to a front opening **73** of the module housing **32.**

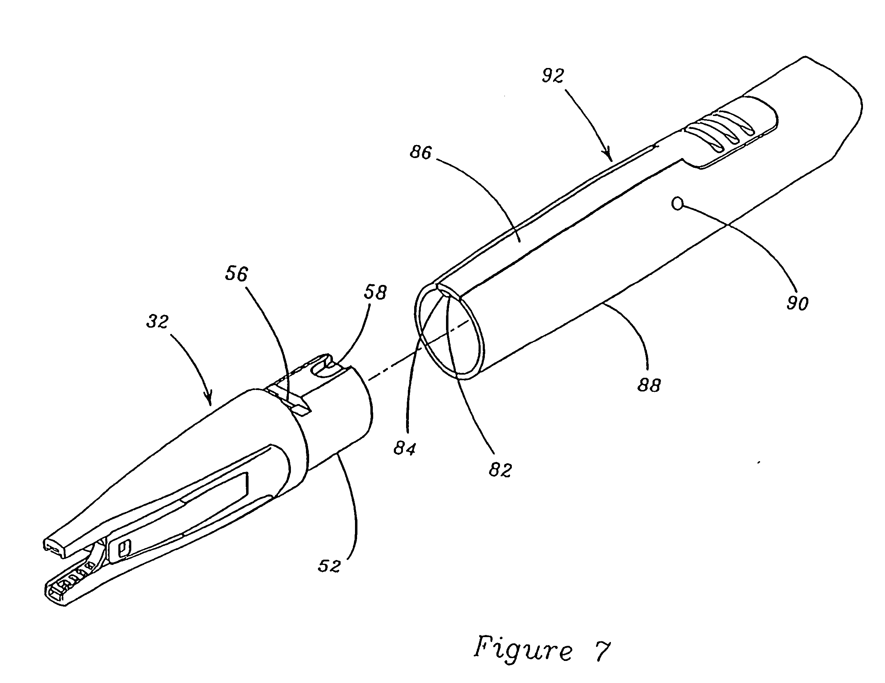

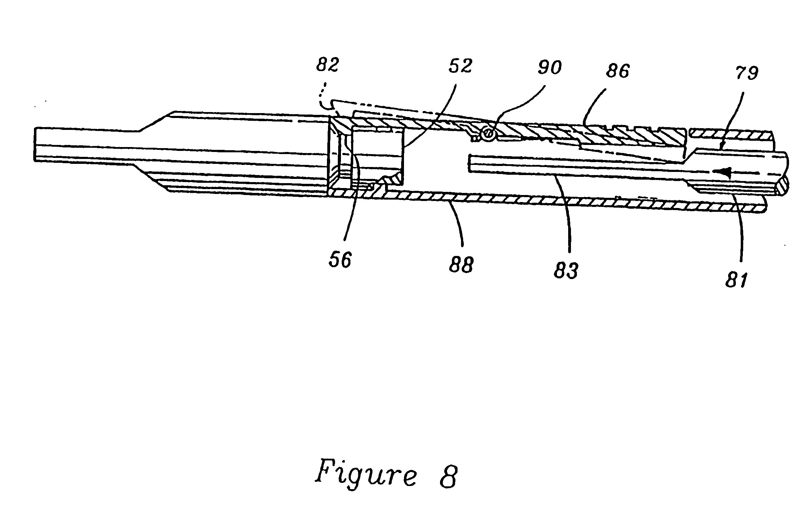

FIG. **7** illustrates the method of attaching the module **23** to the prior art ligation clip applicator **92.** The ligation clip applicator **92** connects to module **23** at a hollow shaft **88** of applicator **92.** This connection is made at a specific orientation controlled by the arrangement of the slot keys **58** that are arranged at diametrically opposed positions around the reduced diameter section **52.** One of the two slot keys **58** must be properly aligned with a key (not shown) formed on the interior surface of the hollow shaft **88** of the ligation clip applicator **92** in order for the module **23** to be attached to the applicator **92.** Improper orientation of the slot key **58** will prevent any connection between the module **23** and the ligation clip applicator **92.** The hollow shaft **88** also includes a spring-loaded latch **86** that is pivotally mounted on a pivot pin **90** carried on the wall of shaft **88.** The distal end of the hollow shaft **88** is provided with a pawl **84.** The reduced diameter section **52** fits into the open distal end of the hollow shaft **88.** To secure the module **23** in the hollow shaft **88,** the proximal end of the spring-loaded latch **86** is pressed down to raise the pawl **84** as shown in phantom in FIG. **8****.** The module **23** is then inserted and the spring-loaded latch **86** released so that the pawl **84** catches the transverse groove **56** in the reduced diameter section **52** to securely lock the module **23** to the distal end of the hollow shaft **88.** Advantageously, the pawl **84** can be provided with a cam surface **82** that rides up on the proximal extreme of the reduced diameter section **52** of the module housing **32.** In this way, the cam surface **82** of the pawl **84** is automatically guided to the groove **56** when the module is inserted into the hollow shaft **88.** Further discussion related to the attachment of the module **23** is found in U.S. Patent No. 5,354,304.

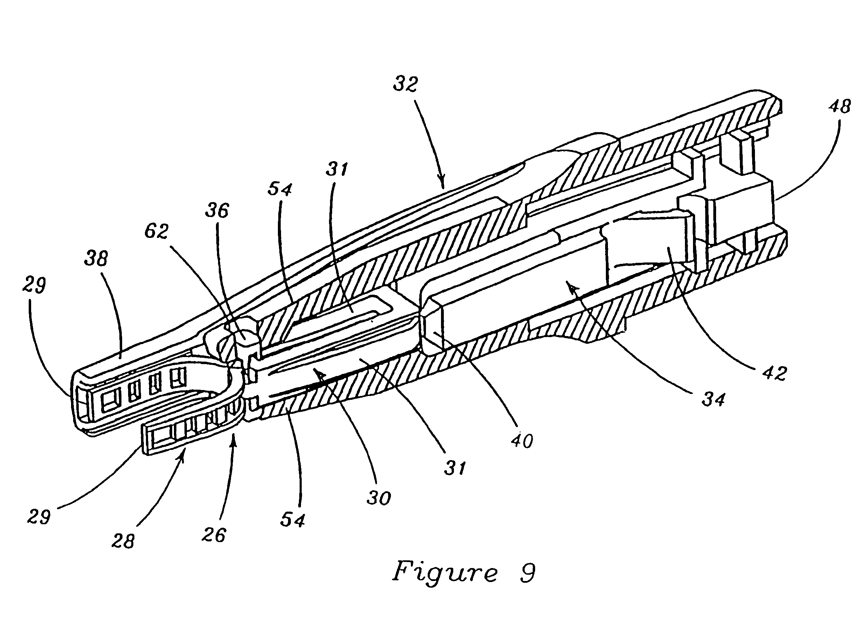

FIG. **9** shows the ligation clip **36** loaded in the module housing **32** and ready to be fired by the ligation clip applicator **92.** The ligation clip **36** is of the type described in U.S. Patent No. 4,590,937 and more specifically in U.S. Patent No. 5,306,283. As explained in detail in '283 patent, the ligation clip **36** is closed by drawing the clip track **28** into the clip body **30** so that the legs **31** of body **30** urge the extensions **29** of the track **28** together. The clip body **30** slides into and is secured within the raceway **80** within the module housing **32.** The raceway **80** is defined by straight ribs on both sides of the module housing **32.** As described briefly above, post **36** extends from the clip track **28** and is shaped to engage in latch holes **62** formed in the distal ends of the integral latches **54.**

In the loaded position shown in FIG. **9****,** the clip body **30** is positioned within the raceway **80** and the Y-shaped clip track **28** is protected within the opposing arms **38** and the extended tips **68** within the module housing **32,** only one of each of which is shown in FIG. **9****.** The opposing arms **38** of the module housing **32** can be used to manipulate tissue when positioning the ligation clip **26** about a vessel to be ligated. The clip track **28** is also held stationary by the post **36** which are engaged in the latch holes **62** in the latches **54** during manufacturing. In the loaded position, the pusher **34** is positioned behind, and in contact with, the clip body **30** and the opposing wings **42** are engaged with the windows **60** in such a manner that withdrawal of the pusher **34** through the rear opening **64** of the module housing **32** is prevented.

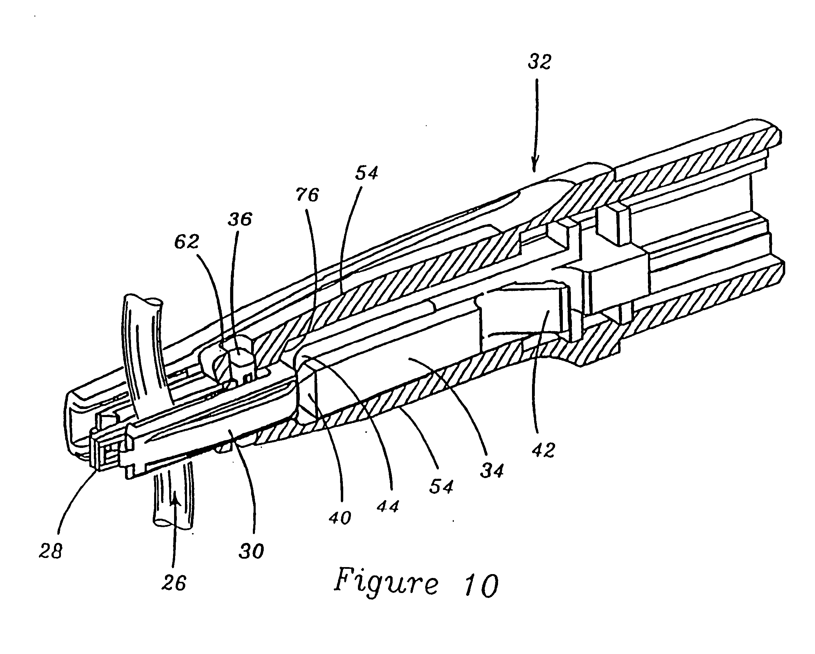

In FIG. **10****,** the ligation clip **26** is shown in a position around the vessel being ligated after the ligation clip applicator **92** has been actuated. During actuation, the cylindrical portion **83** of the ligation clip applicator **92** engages the rear portion **48** of the pusher **34,** thereby forcing the pusher **34** forwardly so that the beveled front end **40** of the pusher **34** contacts the proximal end of the clip body **30** while at the same time engaging the latch ramps **76** of opposing latches **54.** The advancement of the pusher **34** also disengages the opposing wings **42** from the windows **60.** As the pusher **34** travels forward, the opposing wings **42** ride in the racetrack **80.** In this position, the ligation clip **26** is almost fully assembled and the vessel nearly ligated.

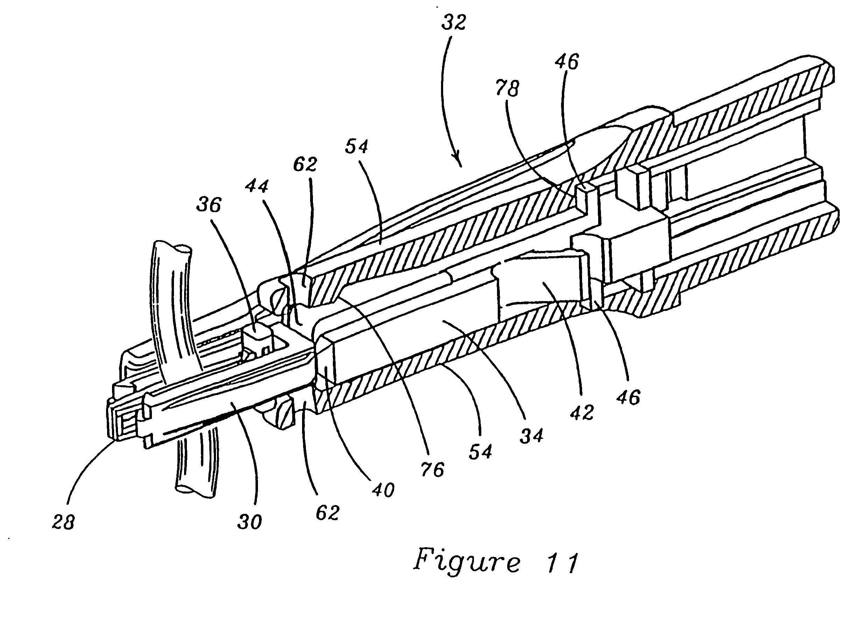

In FIG. **11****,** the ligation clip **26** is now fully assembled and the post **36** is disengaged from the latch holes **62** of the opposing latches **54,** thereby releasing the clip **26** from the module housing **32.** Release is effected by the radiused surfaces **44** of pusher **34** engaging the latch ramps **76** as the pusher **34** is forced forward through the cavity **65** by the cylindrical portion **83.** This engagement of the radiused surfaces **44** against the latch ramps **76** pries open the opposing latches **54** and releases the post **36** of the clip body **30** from the latch holes **62.** During disengagement of the ligation clip **26,** the flanges **46** of the pusher **34** contacts the shoulder **78** of the first larger channel **72,** thereby stopping the forward motion of the pusher **34.**

After release of the ligation clip **26,** the ligation clip applicator **92** may still have sufficient residual actuation remaining in the trigger (not shown) of the applicator **92,** especially if the user does not fully pull the trigger during actuation. This condition places the ligation clip applicator **92** in a "fire-thru" mode, whereby the user can further squeeze the trigger of the applicator **92** and apply residual actuation to the module **23.** In this mode, the cylindrical portion **83** of the applicator **92** will force the pusher **34** further through the second smaller channel **74,** so that the flanges **46** of the pusher **34** are wedged through the smaller opening of the second smaller channel **74** beyond the shoulder **78.**

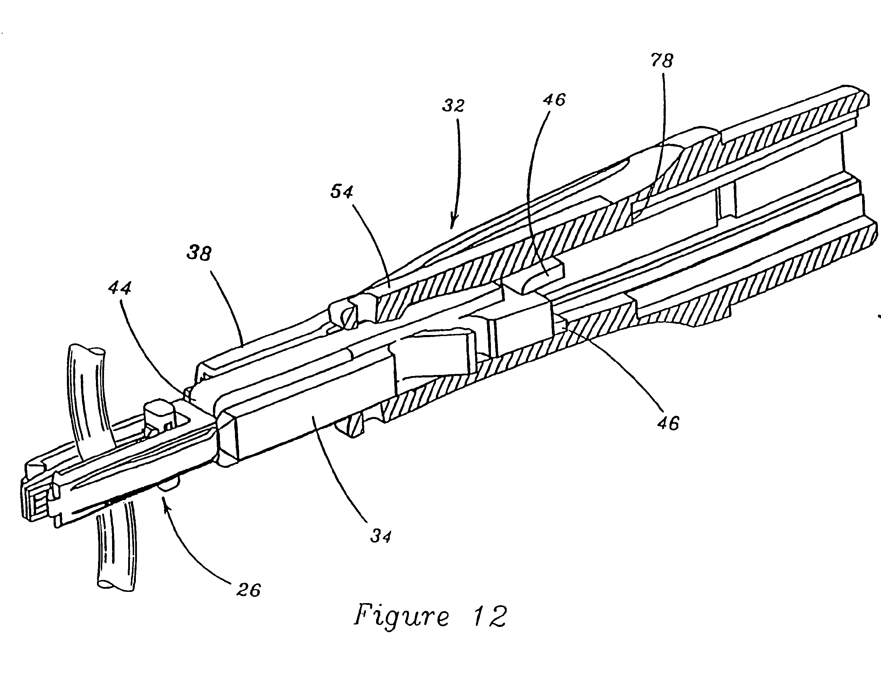

As illustrated in FIG. **12****,** the "fire-thru" mode of the ligation clip applicator **92** causes the flanges **46** of the pusher **34** to bend inward as the flanges **46** are forced through the smaller opening of the second smaller channel **74.** As the pusher is forcibly advanced through the second smaller channel **74,** the bent flanges **46** permanently lodge against the side walls of the channel **74** and halt further advancement of the pusher **34.** Lodging the pusher **34** within the second smaller channel **74** prevents the pusher **34** from being ejected through the front opening **73** of the module housing **32** and causing possible injury to the patient or the user.

It is within the scope of the present invention as defined in the claims that the module **23** can be constructed to any suitable size or configuration commensurate with the description above to fit different types and sizes of ligation clips. Preferably, the module **23** of the present invention is adapted to house and apply either an 8 mm or 12 mm ligation clips used to ligate a vessel.

Although a specific preferred embodiment of the present invention has been described above in great detail, it will be understood that this description is merely for purposes of illustration. Various modifications of and equivalent structures corresponding to the disclosed aspects of the preferred embodiments in addition to those described above may be made by those skilled in the art without departing from the invention as defined in the following claims.

## Claims

1. A module (23) for mounting a two-part ligation clip (26) having a first part (28) formed to be closed about a vessel and a second part (30) movable relative to the first part (28) to cause the first part (28) to close, said module (23) being operable in conjunction with a ligation clip applicator (92) and comprising:
a single piece module housing (32) that includes at least one latch (54) formed as an integral part of the module housing (32), said latch (54) holding the first part (28) of the ligation clip (26) in a stationary position relative to the module housing (32); and
a sliding means, mounted for advancing movement in said module housing (32), for advancing the second part (30) of the ligation clip (26) relative to the first part (28) to close the first part (28), wherein
said sliding means includes a pusher (34) axially slidable in said module housing (32).

2. The module (23) according to claim 1, wherein said pusher (34) includes opposing wings (42) for retaining said pusher (34) in said module housing (32).

3. The module (23) according to claim 1, wherein said module housing (32) is formed with a tapered beveled surface (66), said beveled tapered surface (66) permitting clearer viewing along a longitudinal axis of said module housing (32).

4. The module (23) according to claim 1, wherein said at ' least one latch (54) includes a latch ramp (76), said pusher (34) engaging said latch ramp (76) as said pusher (34) is axially slid through said module housing (32), said latch (54) releasing said first part (28) of the ligation clip (26) when said pusher (32) engages said latch ramp (76).

5. The module (23) according to claim 1, wherein said module housing (32) is formed with opposing arms (38) for embracing at least a portion of the first part (28) of the ligation clip (26).

6. The module (23) according to claim 4, wherein said opposing arms (38) terminate in opposing inwardly directed hooks (68) for embracing at least a portion of the first opposing inwardly directed hooks (68) for manipulating tissue to be ligated.

7. The module (23) according to claim 1, wherein said at least one latch (54) is adapted to grip the first part (28) of the ligation clip (26), the first part (28) of the ligation clip (26) having at least one transversely projecting post (36) and wherein said at least one latch (54) is formed with a hole (62) for receiving the post (36) thereby to provide said gripping action.

8. A module (23) according to any one of the preceding claims and a two-part ligation clip (26) having a first part (28) formed to be closed about a vessel and a second part (30) movable relative to the first part (28) to cause the first part (28) to close, said module (23) being operable in conjunction with a ligation clip applicator.

9. A ligation clip applicator (92) for applying a two-part ligation clip (26) to a vessel, the ligation clip (26) including a first part (28) formed to be closed about a vessel and a second part (30) movable relative to the first part (28) to cause the first part (28) to close, said applicator (92) comprising:
a module (23) according to any one of claims 1 to 7 for supporting a two-part ligation clip, wherein said housing (32) holds the first part (28) of the ligation clip (26) in a stationary position relative to said applicator; and
an actuating means, mountable with said module (23), for actuating said pusher (34).

## Patentansprüche

1. Modul (23) zum Befestigen einer zweiteiligen Abbindeklammer (26), die einen ersten Teil (28) aufweist, der so geformt ist, dass er um ein Gefäß herum geschlossen wird, und einen zweiten Teil (30) aufweist, der in Bezug auf den ersten Teil (28) beweglich ist, um das Schließen des ersten Teils (28) zu bewirken, wobei das Modul (23) in Verbindung mit einem Abbindeklammer-Applikator (92) betätigbar ist und umfasst:
ein Modulgehäuse (32), das mindestens einen als einstückigen Teil des Modulgehäuses (32) ausgebildeten Schnappverschluss (54) umfasst, wobei der Schnappverschluss (54) den ersten Teil (28) der Abbindeklammer (26) in einer stationären Position relativ zum Modulgehäuse (32) hält; und
eine Gleitvorrichtung, die zur vorschiebenden Bewegung im Modulgehäuse (32) angebracht ist, um den zweiten Teil (30) der Abbindeklammer (26) relativ zum ersten Teil (28) vorzuschieben und so den ersten Teil (28) zu schließen, wobei
die Gleitvorrichtung einen Schieber (34) umfasst, der axial in dem Modulgehäuse (32) gleitbar ist.

2. Modul (23) nach Anspruch 1, wobei der Schieber (34) gegenüberliegende Flügel (43) umfasst, um den Schieber (34) in dem Modulgehäuse (32) zu halten.

3. Modul (23) nach Anspruch 1, wobei das Modulgehäuse (32) mit einer sich verjüngenden abgeschrägten Oberfläche (66) ausgebildet ist, wobei die abgeschrägte sich verjüngende Oberfläche (66) eine bessere Visualisierung entlang einer Längsachse des Modulgehäuses (32) erlaubt.

4. Modul (23) nach Anspruch 1, wobei der mindestens eine Schnappverschluss (54) eine Schnappverschlussschräge (76) aufweist, der Schieber (34) mit der Schnappverschlussschräge (76) eingreift, wenn der Schieber (34) axial durch das Modulgehäuse (32) geschoben wird, und der Schnappverschluss (54) den ersten Teil (28) der Abbindeklammer (26) freigibt, wenn der Schieber (34) mit der Schnappverschlussschräge (76) eingreift.

5. Modul (23) nach Anspruch 1, wobei das Modulgehäuse (32) mit gegenüberliegenden Armen (38) ausgebildet ist, um zumindest einen Abschnitt des ersten Teils (28) der Abbindeklammer (26) zu umgreifen.

6. Modul (23) nach Anspruch 4, wobei die gegenüberliegenden Arme (38) in gegenüberliegenden nach innen gerichteten Haken (68) enden, um mindestens einen Abschnitt der ersten gegenüberliegenden nach innen gerichteten Haken (68) zu umgreifen und so das abzubindende Gewebe zu manipulieren.

7. Modul (23) nach Anspruch 1, wobei der mindestens eine Schnappverschluss (54) geeignet ist, um den ersten Teil der Abbindeklammer (26) zu greifen, wobei der erste Teil (28) der Abbindeklammer (26) mindestens einen quer hervorstehenden Stab (36) aufweist und wobei der mindestens eine Schnappverschluss (54) mit einem Loch (62) ausgebildet ist, um den Stab (36) aufzunehmen und **dadurch** die Greifwirkung bereitzustellen.

8. Modul (23) nach einem der vorangehenden Ansprüche und eine zweiteilige Abbindeklammer (26) mit einem ersten Teil (28), der ausgebildet ist, um um ein Gefäß herum geschlossen zu werden, und mit einem zweiten Teil (30), der relativ zum ersten Teil (28) beweglich ist, um den Verschluss des ersten Teils (28) zu bewirken, wobei das Modul (23) in Verbindung mit einem Abbindeklammer-Applikator bedienbar ist.

9. Abbindeklammer-Applikator (92) zum Anbringen einer zweiteiligen Abbindeklammer (26) an einem Gefäß, wobei die Abbindeklammer (26) einen ersten Teil (28) aufweist, der so geformt ist, dass er um ein Gefäß herum geschlossen werden kann, und einen zweiten Teil (30) aufweist, der relativ zum ersten Teil (28) beweglich ist, um den Verschluss des ersten Teils (28) zu bewirken, wobei der Applikator (92) umfasst:
ein Modul (23) nach einem der Ansprüche 1 bis 7 zum Tragen einer zweiteiligen Abbindeklammer, wobei das Gehäuse (32) den ersten Teil (28) der Abbindeklammer (26) in einer stationären Position relativ zum Applikator hält; und
eine Betätigungsvorrichtung, die an dem Modul (23) anbringbar ist, um den Schieber (34) zu betätigen.

## Revendications

1. Module (23) pour installer un clip de ligature en deux parties (26) comprenant une première partie (28) formée pour être fermée autour d'un vaisseau et une seconde partie 30 déplaçable relativement à la première partie 28 pour provoquer la fermeture de la première partie 28, ledit module 23 pouvant être amené à fonctionner conjointement avec un applicateur de clips de ligature 92 et comprenant:
un boîtier de module (32) en une seule pièce qui comprend au moins un loquet (54) formé comme une partie intégrale du boîtier de module (32), ledit loquet (54) retenant la première partie (28) du clip de ligature (26) dans une position stationnaire relativement au boîtier de module (32); et
un moyen de coulissement installé en vue d'un mouvement d'avancement dans ledit boîtier de module (32), pour faire avancer la seconde partie (30) du clip de ligature (26) relativement à la première partie (28) pour fermer la première partie (28), où
ledit moyen de coulissement comprend un poussoir (34) apte à coulisser axialement dans ledit boîtier de module (32).

2. Module (23) selon la revendication 1, où ledit poussoir (34) comprend des ailettes opposées (42) pour retenir ledit poussoir (34) dans ledit boîtier de module (32).

3. Module (23) selon la revendication 1, où ledit boîtier de module (32) présente une surface biseautée diminuée (66), ladite surface biseautée diminuée (66) permettant une vue plus claire selon un axe longitudinal dudit boîtier de module (32).

4. Module (23) selon la revendication 1, où au moins un loquet précité (54) comprend une rampe de loquet (76), ledit poussoir (34) venant en prise avec ladite rampe de loquet (76) lorsque ledit poussoir (34) est amené à coulisser axialement à travers ledit boîtier de module (32), ledit loquet (54) libérant ladite première partie (28) du clip de ligature (26) lorsque ledit poussoir (32) vient en prise avec ladite rampe de loquet (76).

5. Module (23) selon la revendication 1, où ledit boîtier de module (32) présente des bras opposés (38) pour enserrer au moins une portion de la première partie (28) du clip de ligature (26).

6. Module (23) selon la revendication 4, où lesdits bras opposés (38) se terminent par des crochets opposés (68) dirigés vers l'intérieur pour enserrer au moins une portion des premiers crochets opposés (68) dirigés vers l'intérieur pour manipuler le tissu à ligaturer.

7. Module (23) selon la revendication 1, où au moins un loquet précité (54) est apte à saisir la première partie (28) du clip de ligature (26), la première partie (28) du clip de ligature (26) ayant au moins un montant (36) faisant saillie transversalement, et où au moins un loquet précité (54) présente un trou (62) pour recevoir le montant (36) afin de réaliser ainsi ladite action de préhension.

8. Module (23) selon l'une des revendications précédentes et un clip de ligature en deux parties (26) ayant une première partie (28) formée pour être fermée autour d'un vaisseau et une seconde partie (30) déplaçable relativement à la première partie (28) pour provoquer la fermeture de la première partie (28), ledit module (23) étant actionnable conjointement avec un applicateur de clips de ligature.

9. Applicateur de clips de ligature (92) pour appliquer un clip de ligature en deux parties (26) à un vaisseau, le clip de ligature (26) comprenant une première partie (28) formée pour être fermée autour d'un vaisseau et une seconde partie (30) déplaçable relativement à la première partie (28) pour provoquer la fermeture de la première partie (28), ledit applicateur (92) comprenant:
un module (23) selon l'une des revendications 1 à 7 pour supporter un clip de ligature en deux parties, où ledit boîtier (32) retient la première partie (28) du clip de ligature (26) dans une position stationnaire relativement audit applicateur; et
un moyen d'actionnement, pouvant être monté sur ledit module (23) pour actionner ledit poussoir (34).
